# NEW EUROPEAN PATENT SPECIFICATION

(11) **EP 1 309 677 B2**
(45) Date of publication and mention of the opposition decision: **11.04.2012**
(45) Mention of the grant of the patent: 09.07.2008
(21) Application number: 01957519.0
(22) Date of filing: 10.08.2001
(51) Int. Cl.: C12N 15/00

(54) **BACILLUS TRANSFORMATION, TRANSFORMANTS AND MUTANT LIBRARIES**
TRANSFORMATION VON BACILLUS, TRANSFORMANTEN UND MUTANTEN-BIBLIOTHEKEN
TRANSFORMATION DE BACILLE, TRANSFORMANTS ET BIBLIOTHEQUES DE MUTANTS

(30) Priority: 11.08.2000 US 224948 P
(43) Date of publication of application: 14.05.2003
(73) Proprietor: GENENCOR INTERNATIONAL, INC., Palo Alto, California 94304 (US)
(72) Inventor: DIAZ-TORRES, Maria, R., Los Gatos, CA 94134 (US); SCHELLENBERGER, Volker, Palo Alto, CA 94303 (US); SELIFONOVA, Olga, V., Plymouth, MN 55447 (US); MORRISON, Thomas, B., Palo Alto, CA 94306 (US); LEE, Edwin, W., San Francisco, CA 94134 (US)
(74) Representative: Kiddle, Simon John
(86) International application number: PCT/US2001/025166
(87) International publication number: WO 2002/014490

(56) References cited:
- EP-A- 0 761 815
- EP-A- 0 761 815
- WO-A-00/58517
- WO-A-97/20078
- WO-A1-97/16546
- US-A- 4 828 994
- US-A- 5 882 888
- US-A- 5 882 888
- US-A- 6 083 718
- MELNIKOV ALEXANDRE ET AL: "Random mutagenesis by recombinational capture of PCR products in Bacillus subtilis and Acinetobacter calcoaceticus" NUCLEIC ACIDS RESEARCH, vol. 27, no. 4, 15 February 1999 (1999-02-15), pages 1056-1062, XP002355311 ISSN: 0305-1048
- VIDAL MARC ET AL: "Selectable marker replacement in Saccharomyces cerevisiae" YEAST, vol. 10, no. 2, 1994, pages 141-149, XP002355312 ISSN: 0749-503X
- IOANNA P. PETROUNIA ET AL.: "Designed evolution of enzymatic properties" CURRENT OPINION IN BIOTECHNOLOGY, vol. 11, 2000, pages 325-330,
- DOUWE VON SINDEREN ET AL.: "Molecular cloning and sequence of comK, a gene required for genetic competence in Bacillus subtilis" MOLECULAR MICROBIOLOGY, vol. 11, no. 4, 1994, pages 695-703,
- J. HAHN ET AL.: "Regulatory inputs for hte synthesis of comK, the competence transcription factor of Bacillus subtilis" MOLECULAR MICROBIOLOGY, vol. 21, no. 4, 1996, pages 763-775,
- HAHN ET AL: 'Regulatory inputs for the synthesis of ComK, the competence transcription factor of Bacillus subtilis' MOLECULAR MICROBIOLOGY vol. 21, no. 4, 01 January 1996, pages 763 - 775
- GUEROT-FLEURY ET AL: 'Plasmids for ectopic integration in Bacillus subtilis' ELSEVIER SCIENCE - GENE vol. 180, pages 57 - 61
- SHAFIKHANI ET AL: 'Generation of large libraries of random mutants in Bacillus subtilis by PCR-based plasmid multimerization' BIOTECHNIQUES vol. 23, 01 August 1997, pages 304 - 310

## Description

### BACKGROUND OF THE INVENTION

### 1. Field of the Invention

The present invention relates to Bacillus transformation, transformants, and mutant libraries.

### 2. Background

A widely used known method for altering the chromosome of Bacillus involves building plasmid constructs and transforming them into E. coli. Subsequently, the plasmids are isolated from E. coli and transformed into Bacillus. Widespread use of this method can be attributed, at least in part, to the notion that E. coli is easier to transform than Bacillus. In this regard, the *in vitro* ligation of plasmids results in nicked products that can transform E. coli but do not transform Bacillus.

The conventional approach to constructing libraries in Bacillus is based on replicating plasmids. Such an approach, unfortunately, is generally associated with a number of disadvantages, including:
1) One needs an antibiotic or other selectable marker to maintain the plasmid in the cells. This is not desirable for production strains and it constrains the choice of screening conditions.
2) Genes on the plasmid are present in multiple copies. This affects gene regulation and expression.
3) Variations in copy number can skew a library, i.e., one may preferentially identify clones with increased copy number instead of improved gene function.

Alternatively, integrating plasmids or vectors may be used. Integrating vectors do not contain an origin of replication and therefore require insertion into the host chromosome to be stably maintained. However, these are not without problems. Integration occurs via a Campbell-type recombination event that results in a duplication of the cloned region at either end of the inserted (now linear) vector. Depending on the position of the integration genes may be disrupted resulting in poor transformation efficiency.

With either method there is still a need to generate sufficient amounts of the desired sequence to effect an efficient transformation. This was usually accomplished by inserting the desired sequence into a shuttle vector that was inserted into E. coli, allowed to replicate to a high copy number, and recovering the amplified DNA. This process could run into problems due to sequence size; the larger the sequence the more difficult it could be to insert and replicate. Additionally, there were sequences that would not insert or replicate in E. coli resulting in a loss of diversity in the DNA library that was being built. Finally, the high copy number of some plasmids/vectors is deleterious to E. coli.

EP 0761815 describes homologous recombination to integrate a SpoV sequence into a B. thuringiensis strain.

The prior art methods failed to reproducibly render cells hypercompetent nor did they generate large libraries easily in Bacillus and other host cells. In order to generate a small library the prior art utilized E. coli to amplify DNA of interest to obtain a sufficient quantity for transformation of host cells. The methods provided herein allows the generation of large libraries in a reproducible manner without the use of E. coli.

Thus, there is a need for a Bacillus transformation method that is relatively straightforward, efficient and reproducible. In particular, a method is needed that permits the efficient transformation of Bacillus, without requiring intervening steps involving the use of additional microorganisms, such as E. coli. Particularly advantageous would be a transformation method that is amenable to the construction of mutant libraries, and which avoids or overcomes one or more of the above-mentioned disadvantages.

### SUMMARY OF THE INVENTION

The present invention provides methods for building polynucleotide constructs *in vitro,* directly transforming such constructs into super-competent Pxyl-comK strain of Bacillus with good efficiency, and generating populations of mutants (e. g., a mutant library), as set out in the claims.

In one of its aspects, the present invention provides a method of producing a transformed microorganism. According to one embodiment, the method includes the steps of:
(i) selecting a super-competent Pxyl-comK strain of Bacillus;
(ii) producing a polynucleotide construct *in vitro* wherein said DNA construct comprises an incoming sequence of interest, flanked on each side by a homology box, wherein a) said homology boxes are flanked by non-homologous sequence, or b) the assembly of said incoming sequence and said homology boxes is multimerized; and
(iii) directly transforming the microorganism with the construct such that the construct becomes integrated into a chromosome of the microorganism.

In one embodiment, the construct includes mutagenized DNA.

According to one embodiment, the construct is a non-plasmid DNA construct.

The competent microorganism of the genus Bacillus is ultra competent Pxyl-comK strain of Bacillus.

In accordance with one embodiment, the above method additionally includes the steps of (i) selecting a target region in a chromosome of the competent Bacillus, and (ii) increasing (e. g., maximizing) the homology between the target region and the construct.

Also described herein is a library of mutants produced by the above method.

A further aspect of the present invention provides a method for the directed evolution of a sequence in the Bacillus chromosome. One embodiment of the method includes the steps of:
(i) *in vitro* mutagenesis of a DNA construct as described above,
(ii) direct transformation of the mutagenized sequence into a super-competent Pxyl-comK, strain of Bacillus.
(iii) screening for, or selection of, mutants possessing or exhibiting a desired property; and
(iv) repeating steps (i)-(iii) for one or more rounds.

Advantageously, the methods disclosed herein allow one to evolve single-copy genes of a competent Bacillus strain.

In another of its aspects, the present invention provides a method for constructing a polynucleotide sequence in a target locus of a selected recipient strain, wherein the strain includes a selectable marker residing at the target locus. One embodiment of the method includes the steps of:
(i) assembling a construct comprising a sequence of interest, a selectable marker that differs from the residing marker of the recipient strain, and two flanks which are homologous to sequences of the target locus; wherein a) the construct further comprises non-homologous outer flanking sequences or b) the assembly of said incoming sequence, selectable marker and flanking sequence is multimerized;
(ii) directly transforming the recipient strain with the construct under conditions permitting the incoming sequence and selectable marker to replace the residing marker, and selecting for transformants that include the incoming selectable marker;
(iii) repeating steps (i)-(iii), with the newly inserted selectable marker acting as the residing marker,
wherein the selected recipient strain is a super-competent Pxyl-comK strain of Bacillus.

Optionally, after step (ii) the following additional step can be performed: testing the transformants for loss of the residing marker, thereby verifying that the construct was incorporated into the correct locus of the chromosome.

These and other features, aspects and advantages of the present invention will become apparent from the following detailed description, in conjunction with the appended claims.

### DESCRIPTION OF THE DRAWINGS

Figure 1 is a schematic diagram showing cloning by *in vitro* assembly and transformation of competent Bacillus, in accordance with the present invention.
Figure 2 illustrates, in schematic fashion, the addition of non-homologous flanks to the assembled sequences to increase transformation efficiency, in accordance with the present invention.
Figure 3 is a schematic diagram illustrating PCR mutagenesis of a region of the Bacillus chromosome, in accordance with the present invention.
Figure 4 is a schematic diagram illustrating that maximizing the homology between the transforming DNA and the target region of the chromosome can increase the transformation efficiency, as taught by the present invention.
Figure 5 illustrates, in schematic fashion, using a competent host that carries an inactive version of the marker gene, used to select transformants, as taught by the present invention.
Figure 6 shows representative structures of transforming DNA, according to the teachings herein. At top, homology boxes flank an incoming sequence. At center, other non-homologous sequences are added to the ends. At bottom, the ends are closed such that the transforming DNA forms a closed circle or loop.
Figure 7 illustrates, in schematic fashion, Bacillus strain construction by iterative marker replacement, in accordance with the teachings of the present invention.
Figures 8A & B: Figure 8A is a schematic illustration of the DNA construct used in Example 5 wherein the homology box length was varied. Figure 8B is a graph illustrating that PCR fragments containing the gene of interest, a selectable marker and varying lengths of flanking chromosome can be used for transformation directly into Bacillus (crosses), cloned into a plasmid and used for transformation either as an uncut plasmid (closed circles) or a linear plasmid (open circles).
Figure 9 is a schematic illustration of the mutagenized DNA fragment used in Example 2. It is 6.8kb long comprising a left homology box (approx. 2.2 kb), the gene of interest and selectable marker (approx. 2.4 kb), and a right homology box (approx. 2.1 kb).
Figure 10 is a schematic of a three piece PCR fusion construct. The figure also shows the location where the primers align with a sequence within the DNA construct.
Figure 11 depicts an exemplary method of adding nonhomologous flanks to the DNA construct. The DNA construct is inserted into a plasmid, amplified and cut with restriction enzymes to add non-homologous flanking regions.
Figure 12 is a representation of a vector useful in the present invention. In this vector two Bbs I sites have been engineered into the vector. Bbs I is a type IIs restriction enzyme. Other type lis enzyme site may be engineered into the vector instead of the Bbs I site. Thus, the Bbs I site is illustrative and not limitative. The vector is cut with Bbs I and the DNA construct is inserted into the vector.
Figure 13 is a schematic of the process used to prepare the insert that was subsequently ligated into the vector.
Figure 14 is a photograph of a gel showing that the ligation reaction produced large molecular weight ligation products. The gel is a 1.2% agarose gel. Lane 1 was loaded with 2 ul of the ligation product. Lane 2 was loaded with 2 ul of the linearized vector (i.e, the vector digested with Bbs I). Lane 3 contained 250 ng of Roche ladder X standard molecular weight markers.
Figure 15 depicts the modification of a gene of interest. In the figure the MetB gene is modified so that 621 bp are deleted. The full length metB is 672 bp and thus this is not a full gene deletion. The primer N1, N2, N3 and N4 are shown with their relative alignment positions.

### DETAILED DESCRIPTION OF THE INVENTION

The present invention provides methods for building DNA constructs *in vitro,* transforming such constructs into super-competent Pxyl-comK Bacillus strains with good efficiency, and generating populations of mutants.

### Definitions:

Unless defined otherwise herein, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this invention. Although any methods and materials similar or equivalent to those described herein can be used in the practice or testing of the present invention, the preferred methods and materials are described. Numeric ranges are inclusive of the numbers defining the range. Unless otherwise indicated, nucleic acids are written left to right in 5' to 3' orientation; amino acid sequences are written left to right in amino to carboxy orientation, respectively. The headings provided herein are not limitations of the various aspects or embodiments of the invention which can be had by reference to the specification as a whole. Accordingly, the terms defined immediately below are more fully defined by reference to the specification as a whole.

### Transforming DNA or DNA construct

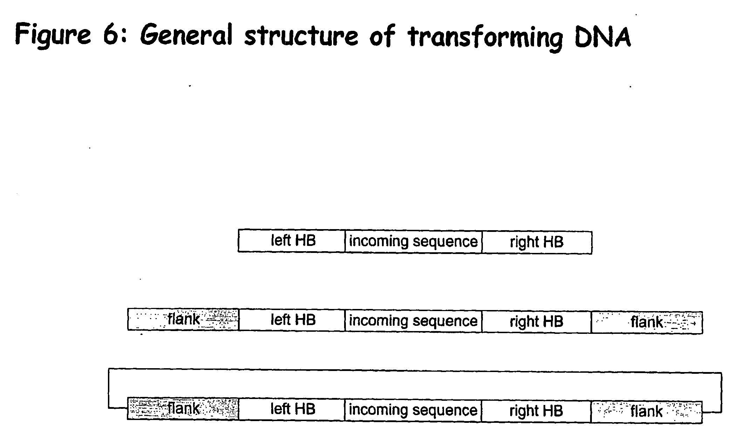

The transforming sequence or transforming_DNA is generated *in vitro* by PCR or other suitable techniques. The typical structure of transforming DNA is shown in a schematic form in Figure 6. The transforming DNA comprises an incoming sequence flanked by homology boxes. In one embodiment, the transforming DNA may comprise other non-homologous sequences, added to the ends, i.e., stuffer sequences, or flanks. The ends can be closed such that the transforming DNA forms a closed circle.

Transforming DNA is DNA used to introduce sequences into a host cell or organism. The DNA may be generated *in vitro* by PCR or any other suitable techniques. In a preferred embodiment, mutant DNA sequences are generated with site saturation mutagenesis in at least one codon. In another preferred embodiment, site saturation mutagenesis is performed for two or more codons. In a further embodiment, mutant DNA sequences have more than 40%, more than 45%, more than 50%, more than 55%, more than 60%, more than 65%, more than 70%, more than 75%, more than 80%, more than 85%, more than 90%, more than 95%, or more than 98% homology with the wild-type sequence. Alternatively, mutant DNA may be generated *in vivo* using any known mutagenic procedure such as, for example, radiation, nitrosoguanidine and the like.. The desired DNA sequence is then isolated and used in the methods provided herein.

The transforming sequences may be wild-type, mutant or modified. The sequences may be homologous or heterologous. Transforming sequence and DNA construct may be used interchangeably.

### Incoming sequence

This sequence can code for one or more proteins of interest. It can have other biological function. In many cases the incoming sequence will include a selectable marker, such as a gene that confers resistance to an antibiotic.

An incoming sequence as used herein means a DNA sequence that is newly introduced into the Bacillus chromosome or genome. The sequence may encode one or more proteins of interest. An incoming sequence comprises a sequence that may or may not already present in the genome of the cell to be transformed, i.e., either a homologous or heterologous sequence (defined herein).

In one embodiment, the incoming sequence encodes a heterologous protein, said protein(s) including, but not limited to hormones, enzymes, growth factors. In another embodiment, the enzyme includes, but is not limited to hydrolases, such as protease, esterase, lipase, phenol oxidase, permease, amylase, pullulanase, cellulase, glucose isomerase, laccase and protein disulfide isomerase.

In a second embodiment, the incoming sequence may encode a functional wild-type gene or operon, a functional mutant gene or operon, or a non-functional gene or operon. The non-functional sequence may be inserted into a target sequence to disrupt function thereby allowing a determination of function of the disrupted gene.

### Flanking Sequence

A flanking sequence as used herein means any sequence that is either upstream or downstream of the sequence being discussed, e.g., for genes A B C, gene B is flanked by the A and C gene sequences. The incoming sequence is flanked by a homology box on each side. In a more preferred embodiment, the incoming sequence and the homology boxes comprise a unit that is flanked by stuffer sequence (as defined herein) on each side. A flanking sequence may be present on only a single side (either 3' or 5') but it is preferred that it be on each side of the sequence being flanked.

### Stuffer Sequence

Stuffer sequence means any extra DNA that flanks the homology boxes, typically vector sequences, but could be any non-homologous DNA sequence. Not to be limited by any theory, a stuffer sequence provides a noncritical target for a cell to initiate DNA uptake.

### Wild-type genes

The terms "wild-type sequence," or "wild-type gene" are used interchangeably and refer to a sequence native or naturally occurring in a host cell. The wild-type sequence may encode either a homologous or heterologous protein. A homologous protein is one the host cell would produce without intervention. A heterologous protein is one that the host cell would not produce but for the intervention.

### Mutant genes

The terms "mutant sequence," or "mutant gene" are used interchangeably and refer to a sequence that has an alteration in at least one codon occurring in a host cell's wild-type sequence. The expression product of the mutant sequence is a protein with an altered amino acid sequence relative to the wild-type. The expression product may have an altered functional capacity, e.g., enhanced enzymatic activity and the like.

### Modified genes

The terms "modified sequence" or "modified genes" are used interchangeably and means a deletion, insertion or interruption of naturally occurring nucleic acid sequence. The expression product of the modified sequence may be a truncated protein if the modification is a deletion or interruption of the sequence. The truncated protein may retain biological activity. The expression product of the modified sequence may be an elongated protein if the modification is an insertion into the nucleic acid sequence. An insertion may lead to a truncated protein as the expression product if the insertion results in the formation of a stop codon. Thus, an insertion may result in either a truncated protein or an elongated protein as an expression product.

### Host cell

"Host cell" means a cell that has the capacity to act as a host and expression vehicle for an incoming sequence according to the invention. According to the present invention, "host cell" means the cells of *Bacillus* which are a super-competent Pxyl-comK strain of Bacillus. As used herein, the genus *Bacillus* includes all members known to those of skill in the art, including but not limited to *B*. *subtilis*, *B*. *licheniformis, B*. *lentus*, *B*. *brevis*, *B*. *stearothermophilus, B*. *alkatophilus, B*. *amyloliquefaciens, B. coagulans, B. ciculans, B*. *lautus and B*. *thuringiensis*.

### Homologous sequence

A homologous sequence is a sequence that is found in the same genetic source or species. For example, the host cell strain may be deficient in a specific gene. If that gene is found in other strains of the same species the gene would be considered a homologous sequence.

### Heterologous sequence

A heterologous sequence is a sequence derived from a separate genetic source or species. A heterologous sequence is a non-host sequence, a modified sequence, a sequence from a different host cell strain, or a homologous sequence from a different chromosomal location of the host cell.

### Homology box

Homology boxes flank each side of the incoming sequence. The sequence of each homology box is homologous to a sequence in the Bacillus chromosome. These sequences direct where in the Bacillus chromosome the new construct gets integrated and what part of the bacillus chromosome will be replaced by the incoming sequence.

### Chromosomal integration

This is a process where the incoming sequence is introduced into the Bacillus chromosome. The homology boxes of the transforming DNA will align with homologous regions of the chromosome. Subsequently, the sequence between the homology boxes is replaced by the incoming sequence in a double crossover (i.e., homologous recombination).

### Homologous Recombination

Homologous recombination means the exchange of DNA fragments between two DNA molecules or paired chromosomes (during crossing over) at the site of identical nucleotide sequences. In a preferred embodiment, chromosomal integration is by homologous recombination.

### Target Sequence

A target sequence as used herein means the DNA sequence in the host cell that encodes the sequence where it is desired for the incoming sequence to be inserted into the host cell genome. The target sequence may encode a functional wild-type gene or operon, a functional mutant gene or operon, or a non-functional gene or operon.

### Selectable Markers

Selectable markers are usually genes that confer antibiotic resistance or a metabolic advantage on the host cell to allow cells containing the exogenous DNA to be distinguished from cells that have not received any exogenous sequence during the transformation. A residing selectable marker is one that is located on the chromosome of the microorganism to be transformed. A residing selectable marker encodes a gene that is different from the selectable marker on the transforming DNA construct.

### Sequence of Interest

As used herein, a sequence of interest may be an incoming sequence or a sequence to be generated *in situ.* The terms gene of interest and sequence of interest may be used interchangeably herein.

### Library of mutants

A population of cells which are identical in most of their genome but include different homologues of one or more genes. Such libraries can be used, for example, to identify genes or operons with improved traits.

### Super competent or Hypercompetent

As used herein, hypercompetent means that greater than 1% of a cell population is transformable with chromosomal Bacillus DNA. Alternatively, hypercompetent means that greater than10% of a cell population is transformable with a self-replicating Bacillus plasmid. Preferably, the super competent cells will be transformed at a rate greater than observed for the wild-type or parental cell population. Super competent and hypercompetent are used interchangeably herein.

### Embodiments:

Figure 6 depicts the DNA constructs that find use in the present invention. Briefly, the DNA construct comprises an incoming sequence flanked by homology boxes on each side, i.e., there is a left homology box and a right homology box, and may be referred to as a basic DNA construct. In one embodiment the basic DNA construct further comprises flanking sequences, i.e., stuffer sequences, on each end and may be referred to as a flanked DNA construct. In another embodiment, the flanked DNA construct is circularized and may be referred to as a circular DNA construct. The circular DNA construct may comprise plasmid DNA or it may comprise non-plasmid DNA in the portion represented by a thin line linking the ends of the flanking sequences, i.e., the flanking sequences' free ends should there be no circularization, in Figure 6.

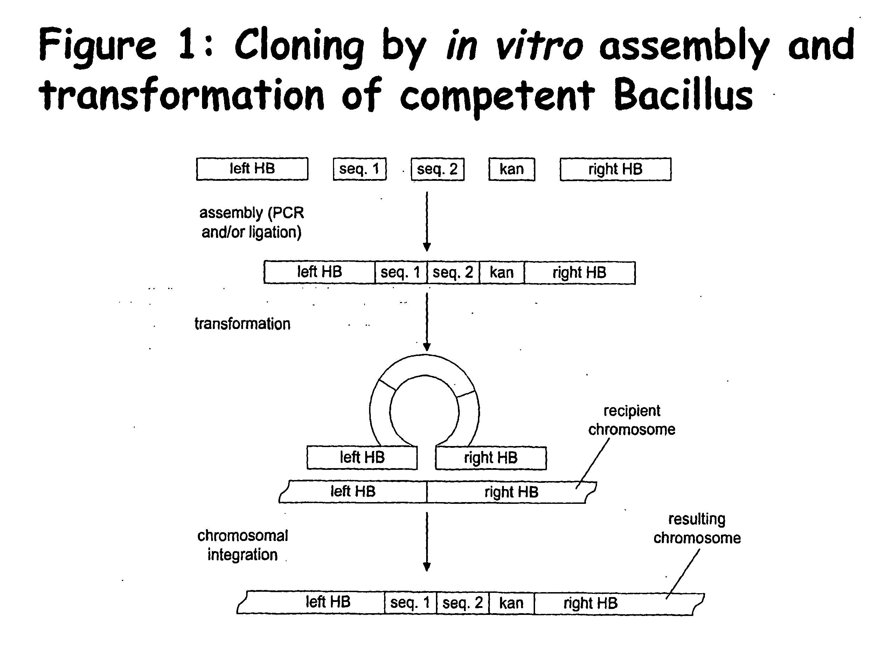

The incoming sequence may encode more than one protein. As shown in Figure 1 the DNA construct comprises a left homology box, an incoming sequence comprising a first sequence (seq. 1) and a second sequence (seq. 2), a selectable marker (here, for example purposes only, the antibiotic marker conferring kanamycin resistance, kan, is used), and a right homology box. It should be noted that the figure is not limiting on the inventive method and that more than two sequences may comprise the incoming sequence, i.e., there may be a third sequence (seq. 3), etc.

The first and second sequences may encode different and distinct proteins, either full length or portions thereof. For example, the first sequence may encode a protease (or portion thereof) and the second sequence may encode a hormone (or portion thereof).

Alternatively, the first and second sequences may encode different portions of the same protein. For example, the first sequence may encode the amino terminal and the second sequence may encode the carboxy terminal of a single protein. This would allow either or both of the sequences to be selectively mutagenized with different mutagenizing protocols being used. Or the carboxy and amino terminal sequences of a protein may be joined while omitting an intervening sequence found in the native protein.

As another option, the first and second sequences may encode variants of a single protein. Thus, for example, sequence 1 may encode Type A hemoglobin while sequence 2 encodes Type S hemoglobin.

The various components of the DNA construct may be assembled by PCR and/or ligation. It should be noted that any technique may be used as long as the DNA construct has the final configuration desired.

Once the DNA construct is assembled *in vitro* it may be used to: 1) insert heterologous sequences into a desired target sequence of a host cell, or 2) mutagenize a region of the host cell chromosome, i.e., replace an endogenous sequence with a heterologous sequence, or 3) delete target genes.

As noted in Figure 1, the recipient chromosome will possess sequences homologous and/or complementary to the homology boxes of the DNA construct. The homology boxes of the DNA construct will align with the homologous region of the recipient chromosome. The DNA construct will then insert into the recipient chromosome, preferably via homologous recombination.

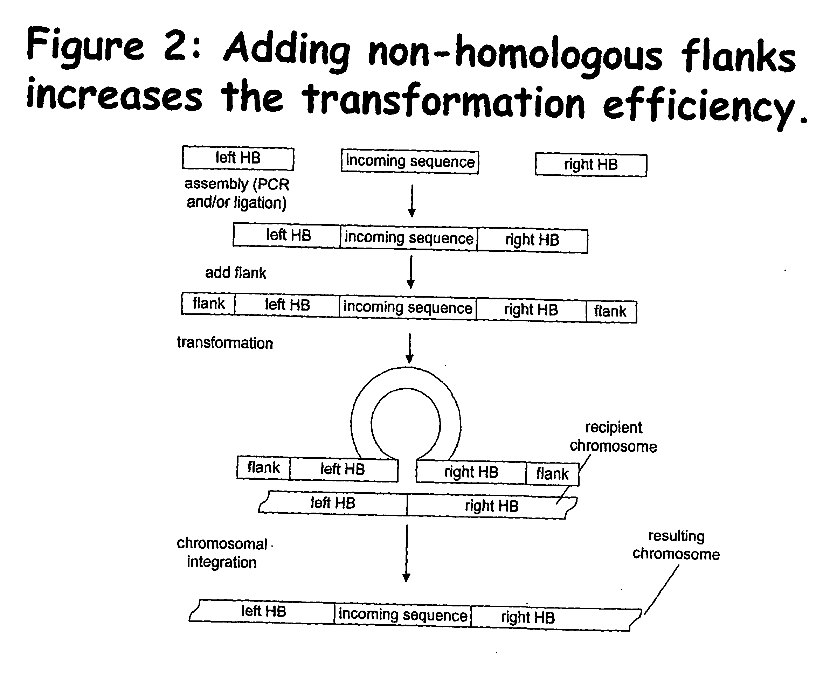

The DNA construct may further comprise flanking, non-homologous sequences, i.e., stuffer sequences, and is illustrated in Figure 2. The addition of non-homologous sequences, as shown below, increases the transformation efficiency. Not to be limited by theory, applicants propose the following mechanism. The mechanism of transformation of competent Bacillus is described in Dubnau, D. (1993) *Bacillus subtilis and other gram-positive bacteria* 555-584. Briefly, the transforming DNA binds to the cell and subsequently one strand is cleaved. The heterologous DNA is taken up by the cell starting from this cleavage site. If the initial cleavage occurs between the homologous flanks (shown in yellow) then chromosomal integration by double crossover becomes impossible. In an embodiment of the present invention, non-homologous flanks are added to the assembled sequences to increase transformation efficiency. Adding flanks to the transforming DNA, as taught herein, increases the probability that the DNA after being taken up will still retain both homologous regions that are required for chromosomal integration. This leads to an increase in transformation efficiency.

In one general embodiment, the present invention involves assembling a DNA construct *in vitro,* followed by direct cloning of such construct into a super-competent Pxyl-comK strain of Bacillus, such that the construct becomes integrated into a chromosome of the Bacillus. For example, PCR fusion and/or ligation can be employed to assemble a DNA construct *in vitro*. In a preferred embodiment, the DNA construct is a non-plasmid DNA construct. In one embodiment, the DNA construct comprises a DNA into which a mutation has been introduced. Bacillus can then be transformed with the DNA construct. In this regard, highly competent mutants of Bacillus are employed to facilitate the direct cloning of the constructs into the cells. Bacillus carrying the comK gene under the control of a xylose-inducible promoter (Pxyl-comK) can be reliably transformed with very high efficiency, according to the teachings herein. In the prior art, Hahn et al, Molecular Microbiology (1996) 21(4), 763-775 describes a Pxyl-comK strain; van Sinderen et al, Molecular Microbiology (1994) 11(4) 695-703 describes comK as a transcription unit required for genetic competence in B subtilis. Direct transformation means that an intermediate cell is not used to amplify, or otherwise process, the DNA construct prior to introduction into the host cell. Introduction of the DNA construct into the host cell includes those physical and chemical methods known in the art to introduce DNA into a host cell without insertion into a plasmid or vector. Such methods include but are not limited to calcium chloride precipitation, electroporation, naked DNA, liposomes and the like. The DNA constructs may be co-transformed with a plasmid without being inserted into the plasmid. A library of mutants can be generated.

Figure 1 illustrates how DNA sequences can be assembled and moved into the Bacillus chromosome, according to the teachings herein. In a preferred embodiment, parts of the assembled sequence are random. As a result, a population of mutants can be obtained, where a single copy of the mutated sequence has been integrated into the Bacillus chromosome.

As previously discussed, a widely used prior method for altering the chromosome of Bacillus involves building plasmid constructs and transforming them into E. coli. Subsequently, the plasmids are isolated from E. coli and transformed into Bacillus. The present invention, in contrast, provides *in vitro* construction and direct transformation into super-competent Pxyl-comK strain of Bacillus, without the use of any such intervening microorganisms.

As also discussed above, the conventional approach to constructing libraries in Bacillus is based on replicating plasmids. Such an approach, unfortunately, is associated with a number of disadvantages, including:
1 One needs antibiotic or other selectable marker to maintain the plasmid in the cells. This is not desirable for production strains and it constrains choice of screening conditions.
2 Genes on the plasmid are present in multiple copies. This affects gene regulation and expression. The approach herein, on the other hand, allows one to evolve single copy genes of a strain.
3 Variations in copy number can skew a library, i.e. one may preferentially identify clones with increased copy number instead of improved gene function.
It will be appreciated that the present invention overcomes such problems associated with the use of replicating plasmids.

### Multimerize the assembled sequence

According to one embodiment of the present invention, the transforming DNA can be multimerized, for example, by ligation. This has a similar effect as adding non-homologous flanks, i.e., stuffer sequences. It increases the probability the DNA after uptake into the cell will still have both homology boxes flanking the incoming sequence, thereby increasing transformation efficiency.

### Mutagenizing a region of the Bacillus chromosome

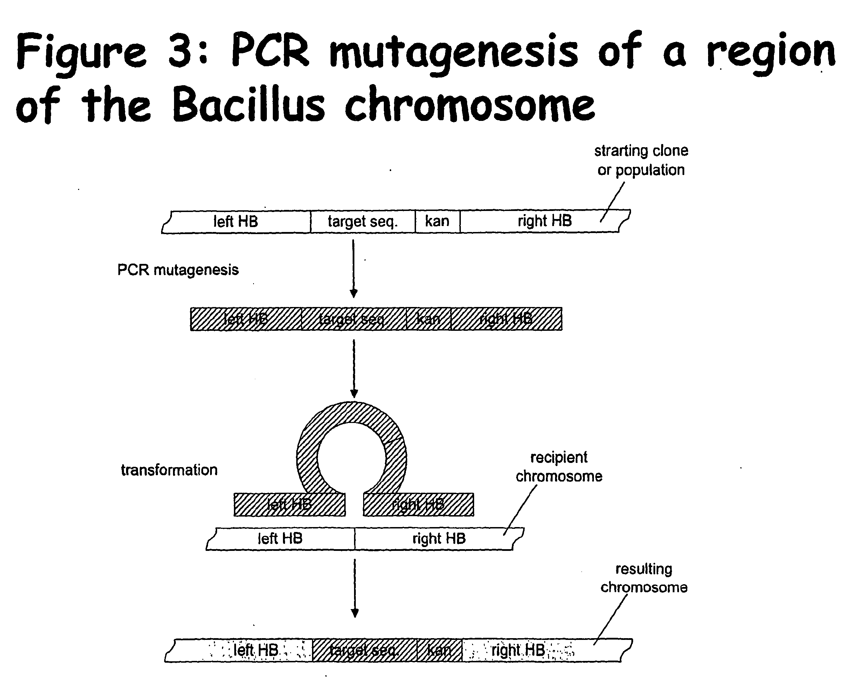

The present invention provides a process for mutagenizing a region of the Bacillus chromosome, an embodiment of which is illustrated in Figure 3 (note, the hatched region has been mutagenized). One can amplify a region of the Bacillus chromosome under mutagenic conditions and transform the resulting DNA back into Bacillus. If the PCR reaction is performed under conditions which favor the introduction of mutations, then one obtains a mutant library. Further, the mutagenic PCR product may be assembled with homology box and insertion sequences to generate transforming DNA in which only the targeted area is mutagenized. To enrich transformants one can introduce a selectable marker close to the target sequence prior to the mutagenesis. Alternatively, if the mutagenized region of the chromosome does not carry a selectable marker, a congression will enrich for cells also taking up transforming DNA. For example, a plasmid bearing a selectable marker is co-transformed with the transforming DNA. The population of cells selected for the plasmid marker will be enriched for the presence of insertion sequences. Later, the plasmid may be removed from the cell, while maintaining the insertion sequence within the chromosome. Lastly, in the absence of selectable marker, the high transformation rate permits direct screening of cells for desired transformants.

In another embodiment, the assembly of long DNA sequences is accomplished *in situ.* Individual DNA constructs are utilized to introduce segments of the final heterologous DNA sequence into a target sequence or locus of the host cell.

### Construction of long sequences by iterative marker replacement

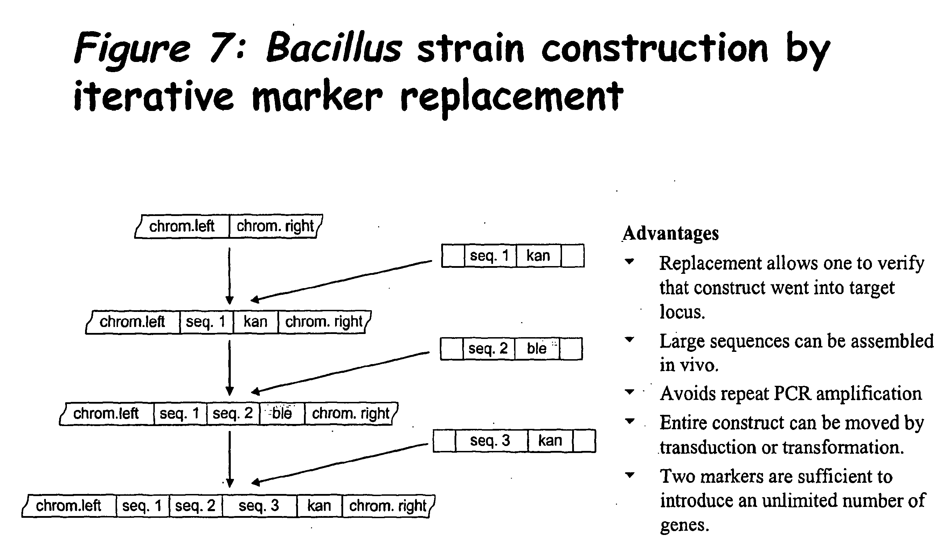

This method, as taught herein and illustrated in Figure 7, provides that one go through several steps of in vitro assembly and transformation. As a result one can introduce many sequences into a particular locus of the Bacillus chromosome. Each round replaces the antibiotic marker that was introduced by the previous round. As a result one can repeat the process many times and still work with only two antibiotic markers.

According to one embodiment, the process comprises the steps of:
(i) by PCR fusion or other suitable technique one assembles a sequence comprising a sequence of interest, a selection marker and two flanks, which are homologous to the target locus;
(ii) the recipient strain is directly transformed with the constructed sequence and one selects for resistance to the incoming selection marker;
(iii) the transformants are then tested for loss of the residing marker which ensures that the construct was incorporated into the correct locus of the chromosome;
(iv) subsequently, the above cycle can be repeated by reversing the role of the incoming and residing markers.

In another embodiment, the microorganism doesn't possess an endogenous selection marker in the first round of transformation and cannot be tested for the loss of a residing marker. Thus, after being transformed the microorganism is screened for the incoming selection marker.

This method allows one to assemble large sequences (e.g., >>5kb) in vivo from smaller pieces, which can be generated in vitro by PCR fusion or other suitable techniques. Only two antibiotic markers are required because each step displaces the marker gene used in the previous round.

The entire resulting construct can be moved between different strains using chromosomal transformation or transduction. Thus, by way of this method, one can accumulate various sequences during the course of a project and retain the ability to simultaneously move them into a new strain.

During the final cloning cycle, one can use a selected gene that is essential for growth under some conditions (e.g., synthesis of an amino acid, utilization of a certain sugar) instead of the incoming marker. The resulting strain would then be free of any antibiotic genes.

It should be appreciated that the iterative aspect of this method generates value as it permits the assembly of large sequences. This method allows one to introduce multiple sequences from various sources into a strain (e.g., bacteria, fungi, eukaryotic, etc.). This method permits one to generate tandem gene repeats as a method for increasing gene copy number. This method permits one to generate strains containing multiple mutations and inserted sequences but no antibiotic markers.

The methods disclosed herein directed to the assembly of transforming DNA constructs may be used to direct the evolution of a sequence or target locus within the host cell.' Selection of the target sequence allows the design and/or *in vitro* mutagenesis of the target sequence. The mutagenesis of a locus of the host cell, i.e., recipient, chromosome is depicted in schematic form in Figure 3. It should be appreciated that although PCR mutagenesis is depicted any in vitro method of mutagenesis may be used. Thus, the depiction of PCR mutagenesis is illustrative and not limitative.

According to one preferred embodiment, the method comprises the following steps:
1) assembling a transforming DNA construct;
2) *in vitro* mutagenesis of the DNA construct;
3) transforming a competent host cell with the mutagenized sequence;
4) screening for or selecting mutants having a desired property or characteristic; and
5) repeating steps 1-4 for one or more rounds.

The host cell is a supercompetent Bacillus carrying the Pxyl-comK construct.

### Identification of Transformants

Although the presence/absence of marker gene expression suggests that the gene of interest is also present, its presence and expression should be confirmed. For example, if the nucleic acid encoding a secretion factor is inserted within a marker gene sequence, recombinant cells containing the insert can be identified by the absence of marker gene function. Alternatively, a marker gene can be placed in tandem with nucleic acid encoding the secretion factor under the control of a single promoter. Expression of the marker gene in response to induction or selection usually indicates expression of the gene of interest as well.

Alternatively, host cells which contain the coding sequence for a sequence o interest and express the protein may be identified by a variety of procedures known to those of skill in the art. These procedures include, but are not limited to, DNA-DNA or DNA-RNA hybridization and protein bioassay or immunoassay techniques which include membrane-based, solution-based, or chip-based technologies for the detection and/or quantification of the nucleic acid or protein.

### Other Embodiments

B. subtilis is a bacteria which is capable of entering sporulation during times of great stress in the environment, such as extreme lack of nutrients. Making this decision triggers a very elaborate and expensive conversion to the sporulation development state. Over 50 genes which need to be expressed for sporulation are under the control of eight sporulation control genes. These are Spo0A, 0B, 0E, 0F, 0H, 0J, 0K, and 0L, with spo0A being the most critical control factor. Mutation in the sporulation control genes allows the cells to ignore their environment so that they fail to enter sporulation and continue production of heterologous or homologous proteins. A mutation in the oppA gene of the oppA operon has been shown to enhance protein production. See WO 00/39323.

The *degU* gene of *Bacillus subtilis* encodes a protein involved in the control of expression of different cellular functions, including degradative enzyme synthesis, competence for DNA uptake and the presence of flagella. Two classes of mutations have been identified in both genes. One class of mutations leads to decreased expression (*degU* mutations) while the second one leads to enhanced expression [degU(Hy) mutations] of regulated genes, i.e., genes regulated by the *degU* system. This second class of mutations is associated with a pleiotropic phenotype which includes the ability to sporulate in the presence of glucose, loss of flagella and decreased genetic competence.

Many industrially important products, e.g., enzymes, hormones, growth factors, and other proteins, are produced from members of the genus Bacilli in large scale fermentation processes. Some of these include proteases, lipases, amylases, and beta-glucanases. The protein of interest to be expressed may be either homologous or heterologous to the host. In the first case overexpression should be read as expression above normal levels in said host. In the latter case basically any expression is of course overexpression. Thus, it is advantageous to have a cell that will fail to sporulate yet possesses enhanced expression of genes of interest.

An oppA (i.e., spo0K) mutation in combination with a degU(Hy) mutation would appear to be ideal for production of a gene of interest. However, it has been shown that mutation of the oppA gene results in a decreased competency. See Rudner et al., J. Bacteriology (1991) 173:1388-1398. As noted above the degU(Hy) mutant also results in decreased competency. Thus, introduction of a gene of interest or other genetic manipulation into such a host cell would be significantly more difficult than in the absence of such mutation.

It has been advantageously found that the inventive methods described herein overcome this difficulty. Use of a pyxl-comK Bacillus strain overcomes the decreased competency exhibited by degU(Hy) oppA⁻ strains. It has been found that the introduction of pyxl-comK into Bacillus not only restores competency but the cells are hypercompetent relative to wild-type (or parental) cells. Thus, heterologous or homologous sequences may be introduced into previously low competency cells.

Transforming Bacillus with PCR-generated DNA and getting many transformants (>100). The methods provided by the present invention allows for the generation of large libraries.

The methods disclosed herein may be used with mutations that enhance competence. Employing other mutations to enhance competence, e.g., comS instead of comK, mutations to comS homologs and the like are contemplated by the present invention.

The methods described herein may be used in any microorganism that can be made competent. Direct transformation in other organisms which can be made competent (like Acinetobacter, Thermus, Deinococcus Radiodurans) is contemplated.

The methods herein should work for any recombination goal, such as insertions, deletions or replacements. Plasmids with temperature sensitive replication would facilitate the curing step. Ligating the PCR products to form concatamers are contemplated for improving the transformation frequency and allowing smaller homology boxes to be used.

### Have inactive homologuereside in the host to improve transformation efficiency

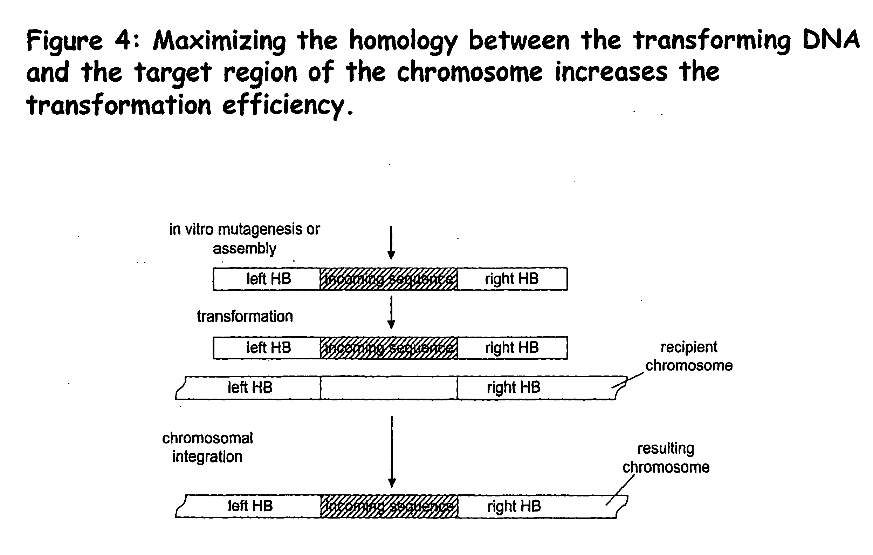

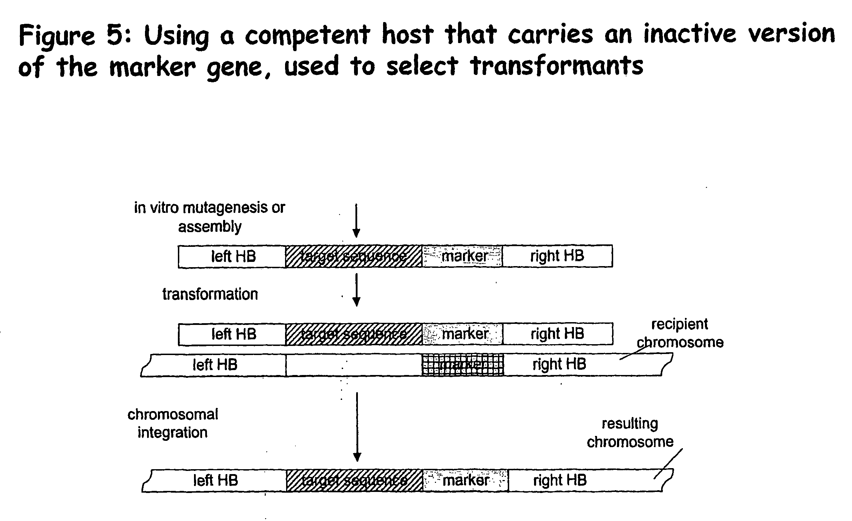

A mutagenesis experiment, in accordance with an embodiment of the present invention, is illustrated in Figure 3. In the illustrated embodiment, the incoming (mutagenized) DNA comprises a sequence which shares no homology with the target area of the Bacillus chromosome. In such case, a successful chromosomal integration requires that both homologous flanks of the incoming DNA align with their respective homologous regions of the Bacillus chromosome. The DNA between the two homologous regions is required to "bulge" if the incoming DNA differs in its length from the target region of the chromosome. As a result, the transformation efficiency is diminished. If the target region of the Bacillus chromosome is made highly homologous to the entire incoming DNA, then the alignment of both sequences becomes more efficient and the overall transformation efficiency can be increased (see Figure 4). One preferred way to implement this concept is to construct a recipient strain which contains a non-functional mutant of a selectable marker (see Figure 5).

### EXAMPLES

The following examples are illustrative and are not intended to limit the invention.

### Example 1:

### Construction of an integrative plasmid containinga xylR- PxyIA- comK cassette (plasmid pMComK1) and transformation into Bacillus.

A fragment containing the *xylR* repressor gene and the *xylA* promoter was obtained by PCR using primers xyIR.2.f (this primer will incorporate a HindIII site) and xyIA.1.r and chromosomal DNA from BG168. A second fragment containing the comK gene including the first aa codon was obtained by PCR using primers comK.2.f and comK.2.r (this primer will incorporate a Xbal site) and same chromosomal DNA. After purification, the fragments were fused together by mixing them in a PCR reaction containing the external primers (xylR.2.f and comk.2.r). A PCR fragment of the expected size was purified, digested with HindIII/XbaI and ligated into the integration vector pJM103 (Kapp, Edwards et al., 1990) (containing carbenicillin and chloramphenicol resistance genes as markers) digested with the same restriction enzymes. Ligation products were transformed into MN296 *E*. coli cells, colonies were selected on 50ug carbenicillin, plasmid DNA was isolated and screened for the 2.1 kbp xylR-PxylA-comK insert by DNA digest. The plasmid was integrated into B. subtilis. The resulting strain was grown overnight in L-broth medium, diluted to 1 OD₆₀₀ in L-broth containing 1% xylose and grown 2 hours with shaking to induce comK expression. The resulting process produced a population of cells in which greater than 1% of cells are transformed by bacillus chromosomal DNA containing a marker, indicating that these cells were super competent. Cells were considered supercompetent if greater than 10% of the cells were transformable with a Bacillus self-replicating plasmid. These cells were utilized in the following examples.

The primer sequences used were as follows:
xylR.2.f (SEQ ID NO: 1)
   GCGCGCAAGCTTTGCTTCAGAAATACTCCTAGAATAAAAAAACTC
xylA.1.r (SEQ ID NO: 2)
   GGTGCGTCTGTTTTCTGACTCATGTGATTTCCCCCTTAAAAATAAATTCA
comK.2.f (SEQ ID NO: 3)
   TGAATTTATTTTTAAGGGGGAAATCACATGAGTCAGAAAACAGACGCACC
comk.2.r (SEQ ID NO: 4)
   GCGCGCTCTAGAGGTATATGGCATCACCGGAGGAATTCCG

### Example 2:

### Mutagenesis of the subtilisin gene using Z-Tag polymerase

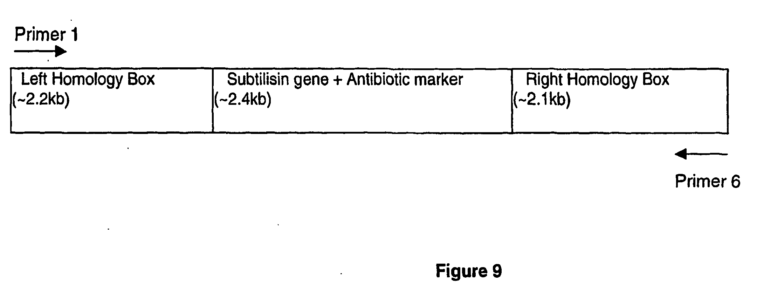

This Example describes an exemplary method to randomly mutagenize a large DNA fragment, containing a gene of interest (e.g. subtilisin gene) with an antibiotic marker and approximately 2kb of homologous DNA on either side of the subtilisin gene. In this specific example, the mutagenized DNA fragment is 6.8kb long comprising a left homology box (approx. 2.2 kb), the gene of interest and selectable marker (approx. 2.4 kb), and a right homology box (approx. 2.1 kb). See Figure 9.

Chromosomal DNA of Bacillus was extracted from an overnight culture of cells grown on semi solid nutrient agar plates (LA) + chloramphenicol plates. Usually three colonies from the overnight plate were resuspended into 0.1 ml of SMM medium (0.5M sucrose, 0.02M sodium maleate, 0.02M magnesium chloride-6H₂O, pH 6.5) containing lysozyme (100,000 U). The cell suspension was incubated for 30 minutes at 37°C with shaking. An additional 1 ml of SMM was added to the cells and the suspension microfuged for 1.5 minutes. The supernatant was removed and the step repeated. Finally the cell pellet was resuspended in 10 mM Tris (pH 8.0) and 0.5 mM EDTA, vigorously vortexed for 30 seconds and the sample was frozen at -20°C.

For PCR mutagenesis, a 100ul PCR reaction was set up using the Z-Taq polymerase kit (TaKaRa Shuzo Co., Ltd.). A typical reaction mixture contained 0.25uM of both primers, 125uM of Z-Taq dNTP mixture, 5-10ng of the chromosomal DNA, 2.5U of Z-Taq polymerase, 1X Z-Taq polymerase buffer. The PCR amplification parameters were: 98°C for 10sec (first cycle only) followed by 98°C for 5sec, 58°C for 10sec 72°C for 2.5 minutes. The PCR reaction was run for a total of 30 cycles. The primer sequences to amplify the 6.8Kb fragment were as follows:
Primer 1 ATATGTGGTGCCGAAACGCTCTGGGGTAAC (SEQ ID NO: 5)
Primer 6 CTTTTCTTCATGCGCCGTCAGCTTTTTCTC (SEQ ID NO: 10)

After the amplification process, the PCR products were analyzed on an agarose gel. For a typical PCR reaction, the limited amount of dNTP used yielded approximately 15ug of DNA. The mutagenized DNA was then transformed into Pxyl-comK Bacillus strains to generate a library.

### Example 3:

### Random mutagenesis of the signal sequence and propeptide of subtilisin

This Example provides an exemplary method for randomly mutagenizing the signal sequence and propeptide of subtilisin.

Primers used in the random mutagenesis reactions were as follows :
1 ATATGTGGTGCCGAAACGCTCTGGGGTAAC (SEQ ID NO: 5)
2 GACTTACTTAAAAGACTATTCTGTCATGCAGCTGCAATC (SEQ ID NO: 6)
3 GATTGCAGCTGCATGACAGAATAGTCTTTTAAGTAAGTC (SEQ ID NO: 7)
4 CTAATTCCCCATGGCACTGATTGCGC (SEQ ID NO: 8)
5 GCGCAATCAGTGCCATGGGGAATTAG (SEQ ID NO: 9)
6 CTTTTCTTCATGCGCCGTCAGCTTTTTCTC (SEQ ID NO: 10)

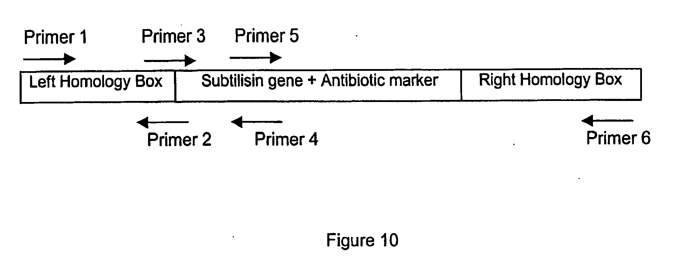

To randomly mutagenize the signal sequence and propeptide of subtilisin gene, a PCR reaction using Primers 1 and 2 generated the 2.2 Kb left flanking region. Primers 3 and 4 were used to mutate a 646bp region comprising of the signal sequence and propeptide region. Primers 5 and 6 were used to generate the 3.9kb right flanking region. Primers 2 & 3 are complementary to one another, as are primers 4 & 5. See Figure 10. A typical amplification reaction (100ul) was set up using either 0.5uM of Primers 1 and 2 (for the 2.2Kb fragment) or 0.5uM of Primers 5 and 6 (for the 3.9Kb fragment) and 200uM of dNTP, 2ul of log phase liquid culture grown to OD₆₀₀=0.5 (source of Bacillus chromosomal DNA), 4U rTth XL polymerase, 1.25U Pfu Turbo DNA polymerase, 1X rTth XL polymerase buffer and 1.1mM Mg (OAc)₂.

The amplification parameters for the 2.2Kb and 3.9Kb fragments were: 95°C for 3min, 95°C for 30sec, 54°C for 30sec, and 68°C for 2min for a total of 30 cycles.

The PCR reaction products were analyzed on an agarose gel. If the correct size fragment was seen then the PCR product was purified using the QIAquick PCR Purification Kit.

The 646bp fragment for mutagenizing the maturation site was amplified using Primers 3 and 4 (0.5uM each), 33ul 3x dNTP, 2ul of liquid culture grown to OD₆₀₀=0.5 (source of Bacillus chromosomal DNA), 0-0.3mM MnCl2 (varies upon the rate of mutagenesis desired), 5.5mM MgCl₂, 5U Taq polymerase, 1X Taq polymerase buffer in a 100ul reaction. The PCR amplification parameters were as follows: 95°C for 30sec, 54°C for 30sec, and 68°C for 30sec for a total of 30 cycles. The PCR reaction products were analyzed on an agarose gel. If the correct size fragment was seen, the PCR product was purified using the QlAquick PCR Purification Kit.

The assembly of the entire 6.8kb fragment containing the mutagenized maturation site was done using 3-5ul each of 646bp, 2.2kb, and 3.9kb fragments, 0.5uM each of Primers 1 and 6, 300uM of dNTP, 4U of rTth XL polymerase, 1.25U Pfu of Turbo DNA polymerase, 1X rTth XL polymerase buffer, and 1.1 mM Mg (OAc)₂ in a 100ul reaction. The parameters for the assembly reaction were as follows: 95°C for 30sec, 48-50°C for 30sec, and 68°C for 7min for a total of 30 cycles. The PCR reaction products were analyzed on an agarose gel. If the correct size fragment was seen, the PCR product was transformed into Pxyl-comK Bacillus strains to generate a library. A total of 9,000 transformants were obtained.

### Example 4:

### Increasing the efficiency of transformationby adding non-homologous flanks to the transforming DNA

This Example provides an exemplary method to increase the transformation efficiency of Bacillus for obtaining larger libraries. Although this example utilizes a plasmid that is amplified in E. coli, one skilled in the art will recognize any method that results in the addition of non-homologous flanks may be used with the present invention. The use of E. coli in the present example was a rapid and simple means for adding non-homologous flanks and should not be construed as limiting.

Figure 9 shows a schematic of the DNA construct used for the present example. Primers 1 and 6 were used to generate the 6.8Kb DNA fragment. A typical PCR reaction (100ul) contained 0.25uM each of Primers 1 and 6, 300uM of dNTP, 5-10ng chromosomal DNA, 2.5U of Pfu Turbo DNA polymerase (Stratagene), and 1.5X of Pfu Turbo DNA polymerase buffer. The PCR amplification parameters were as follows: 95°C for 30sec, 54°C for 30sec, and 68°C for 7min for a total of 30 cycles. The PCR reaction products were analyzed on an agarose gel. If the correct size fragments were seen, the 6.8Kb DNA fragment was cloned into the TOPO vector following the manufacturers protocol (Invitrogen). The vector was then transformed into TOP 10 E. coli competent cells.

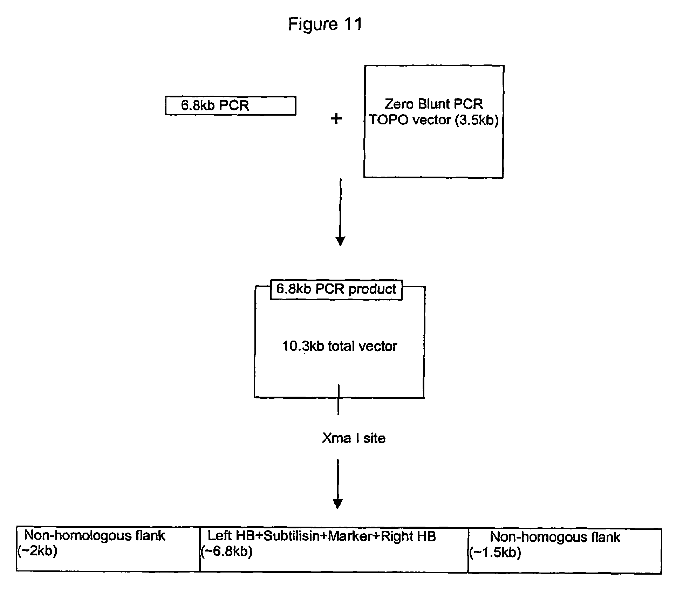

A 10.3Kb fragment was generated as shown in Figure 11. Plasmid DNA was prepared from the transformed E. coli cells using the QIAprep Spin Miniprep to obtain lots of DNA. The plasmid DNA was digested with Xma I restriction endonuclease (no Xma I site is present in the 6.8kb DNA fragment) to linearize the vector.

The non-homologous flanks were derived from the TOPO cloning vector and were of E. coli based plasmid origin; therefore, the sequences were not expected to have any significant homology to regions in the Bacillus chromosome.

Transformation efficiency of Pxyl-comK Bacillus competent cells for the two constructs: without (6.8Kb fragment) and with (10.3Kb fragment) the non-homologous flanking sequences was compared.

Transformation with 2.2x10⁻¹⁴ moles DNA (approximately 1ug/ml) of the 6.8kb DNA fragment (i.e. without the non-homologous flanks) yielded approximately 3.2x10⁴ cfu/ml (0.01 % transformation efficiency). Transformation with 2.2x10⁻¹⁴ moles DNA of the 10.3Kb linearized fragment (i.e. with the non-homologous flanks) yielded approximately 7.2x10⁵ cfu/ml (0.25% transformation efficiency).

As an alternative to using the TOPO cloning kit from Invitrogen, one could also ligate the 6.8kb PCR product to itself. The multimerized DNA can then be transformed into Pxyl-comK Bacillus strains to generate a library.

### Example 5:

### Optimizing Double Cross OverIntegrations by varying the size of the homology box

This Example provides an exemplary method to evaluate transformation efficiency of Bacillus as a function of varying the size of the homology box and stuffer sequence.

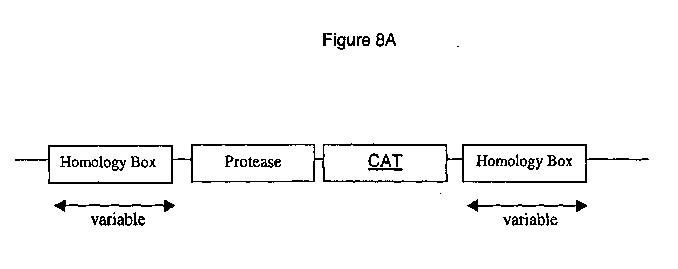

Using primers of varying lengths that contained flanks corresponding to 100, 200, 400, 800, and 1600 bp homology boxes, a series of PCR fragments were generated containing genes coding for a protease, a selectable marker (CAT) and increasing amounts of flanking chromosome sequence. The DNA construct is shown in schematic form in Figure 8A.
The various primers used for the amplification reaction were as follows:

| HB size | Forward Primer | Reverse Primer |
|---|---|---|
| 100 | CCTTGCAAATCGGATGCCTG (SEQ ID NO:11) | CGCTGTTATTGCTTTTGTTTTCT GT (SEQ ID NO: 12) |
| 200 | GTTGGATAGAGCTGGGTAAAG CC (SEQ ID NO: 13) | CGCCGGATTTTATGTCATTGATA A (SEQ ID NO: 14) |
| 400 | AGCCGTTTTGCTCATACAAGC TT (SEQ ID NO: 15) | TGAAGTGAACATGTCAGAAA (SEQ ID NO:16) |
| 800 | ATAGCTTGTCGCGATCACCT (SEQ ID NO:17) | TTTTTGCAGACCGTTGGTTT (SEQ ID NO:18) |
| 1600 | CGCGACACAGCAGTTCAGCA (SEQ ID NO:19) | TATCATTTTTGCTTAATTTG (SEQ ID NO:20) |

A typical PCR reaction (100ul) contained 0.25uM of Forward and Reverse Primers each, 300uM of dNTP, 5-10ng 6.8Kb DNA fragment generated in Example 4, 2.5U Pfu Turbo DNA polymerase, and 1.5X Pfu Turbo DNA polymerase buffer. The cycling conditions for producing DNA fragments with different sized homology boxes were as follows: 95°C, 30 sec; 52°C, 30 sec, and 68°C for 3 to 6 minutes for a total of 30 cycles (extension times depended on the expected product length, the rule being 1000 bp/min).

An aliquot of this reaction was saved for the direct transformation into Bacillus, while the rest was cloned into the Zero Blunt TOPO vector following manufacturer directions (Invitrogen). The cloned fragments were transformed into competent *E. coli* cells and plasmid DNA prepared.

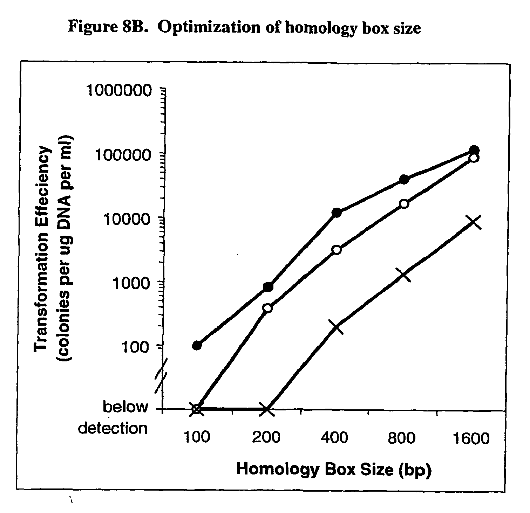

Figure 8B shows the transformation efficiency for various sized homology boxes in either uncut plasmid, linear plasmid or PCR product (no plasmid). Transformation efficiency increases as the homology box size increases for each DNA construct tested. 0.2 ug of uncut plasmid **(closed circle),** linear plasmid (sfil at 50C for 5 hrs, **open circle**), or the PCR products (direct transformation, cross) were transformed into 0.2 ml competent OS22.9 Bacillus cells and colonies on solid L-agar plates with 10 ug/ml chloramphenicol were counted in order to estimate transformation efficiency. To confirm that the majority of transformants were double cross over integrations, chromosome DNA from twenty randomly selected clones was amplified using primers flanking the homology box, these products indicated the selected clones had inserts generated by double crossover.

The effect of homology box size on transformation efficiency was measured (Figure 8B). Transformation efficiency was proportional to homology box size.

### Experimental Discussion

Part of the improvement was due to having a larger length of DNA because the efficiency of transformation jumped over 10-fold when the PCR fragment was cloned into a vector. By cloning into a vector, the integrating DNA is flanked by stuffer sequence; presumably this stuffer sequence reduces the chance that the Bacillus DNA transporter will initiate in sequences between the homology boxes.

### Example 6:

### Site Directed Mutagenesis using QuikChange

This example describes an exemplary method to perform site directed mutagenesis on the gene of interest and directly transform Bacillus strains with the mutagenized DNA.

Site-saturation libraries were created by PCR at 3 different sites in the gene of interest (in this case protease) by using QuikChange (Stratagene)

The primers used were as follows:
Primer A: GAAGAGGATGCAGAANNSACGACAATGGCGCAATC (SEQ ID NO: 21)
Primer B: GATTGCGCCATTGTCGTSNNTTCTGCATCCTCTTC (SEQ ID NO: 22)
Primer C: GAGGATGCAGAAGTANNSACAATGGCGCAATCAG (SEQ ID NO: 23)
Primer D: CTGATTGCGCCATTGTSNNTACTTCTGCATCCTC (SEQ ID NO: 24)
Primer E: GATGCAGAAGTAACGNNSATGGCGCAATCAGTG (SEQ ID NO: 25)
Primer F: CACTGATTGCGCCATSNNCGTTACTTCTGCATC (SEQ ID NO: 26)

Three separate PCR reactions were set up using primer pairs A&B, C&D and E&F. A typical PCR reaction (100ul) contained 1X Pfu Buffer, 1.5ul 10mM dNTPs, 1ul of 25uM primer, 1ul Pfu Turbo DNA polymerase, 200ng of plasmid DNA. The cycling conditions were: 95°C for 35 seconds for one cycle; (95°C for 35 seconds, 50°C for 1 minute, 68°C for 16.5 minutes) for 16 cycles, and 68°C for 7 minutes

The expected 7.8Kb band was identified on the agarose gel (~100ng/ul). The PCR products were digested with 1 uL Dpnl at 37C for an hour to eliminate the pMEO3 template. The digestion reaction was spiked with another 1ul of Dpnl and digested for another hour. A mock PCR reaction that did not undergo the PCR amplification was also digested to see how well Dpnl works to get rid of the template DNA (template control).

A supercompetent Bacillus strain was directly transformed with the digested products. About 200ng of the library was incubated with 100ul of OD 600=0.5. The reaction was incubated at 37°C for 1.5 hours with shaking. Two transformations were set up for each of the conditions, which included the three-mutagenesis reactions (with A&B, C&D, and E&F), a template control, a parent vector control and no DNA condition. Serial dilutions of the cell suspension were streaked on selection plates and following O/N incubation; the transformation efficiency was computed from the number of colonies obtained.

Transformation efficiency as follows:

| DNA source | Colonies/ug |
|---|---|
| A&B | 280 |
| C&D | 305 |
| E&F | 405 |
| Template control | 0 |
| Parent vector | 2.50E+05 |
| No DNA | 0 |

### Example 7:

### Direct transformation of ligated product.

This example provides an exemplary method of mutagenizing the gene of interest with error prone PCR (forms separate PCR products which can be annealed together) and directly transforming the ligated product into Bacillus strain.

### Generation of the vector

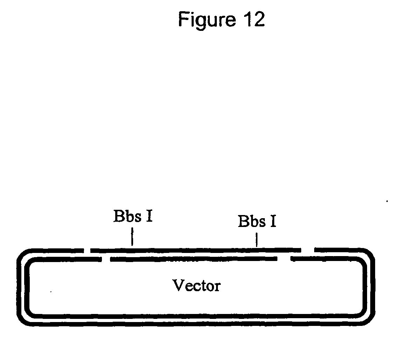

The source of the vector DNA was the 800bp homology box plasmid described in Example 5. Bbs I sites were incorporated into this vector and 20ug of the plasmid was digested overnight at 37°C in New England Biolabs Buffer 2 with Bbs I to generate the vector with flanking sites. See Figure 12.

### Preparation of insert

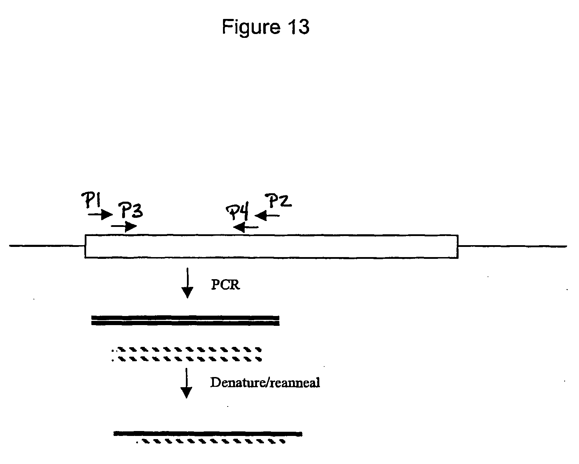

Insert DNA was generated from annealing two overlapping error prone PCR products. See Figure 13. The primer sets used for the PCR were:
P1 CTCTGAATTTTTTTAAAAGGAGAGGGTAAAG (SEQ ID NO: 27)
P2 AATTCCCCATGGTACCGATTGCG (SEQ ID NO: 28)
P3 TCTACTCTGAATTTTTTTAAAAGGAGAGGGTAAAG (SEQ ID NO: 29)
P4 CCCCATGGTACCGATTGCG (SEQ ID NO: 30)

Error prone PCR products were formed by both sets of primers (P1&P2 [solid line product] and P3&P4 [hatched line product]) using conditions described in Example 3, with cycling conditions 94°C for 1 min, 50°C for 1 min, 68°C for 2min, for 30 cycles. Negative control was a reaction without MnCl₂. PCR products (330bp) were purified using Qiaquick PCR columns and pure DNA was pooled together.

For annealing of the two products, 1.3ug of each was combined and heated at 95°C for 5 min then allowed to cool to room temp in heat block. Only one out of four annealed products were expected to ligate properly with vector correctly.

### Ligation

A 1:5 Molar ratio (vector:insert) was used for ligation (DNA ligation kit from PanVera TAK6021) with a total of 440ng of DNA in the reaction mixture (10ul of vector+ insert DNA + 10ul of Takara Biomedicals Ligase solution). This 1:5 ratio was to the insert of interest (1 out of 4 of the reannealed products) so overall it was actually a 1:20 ratio (vector:annealed PCR product). Appropriate DNA controls were also made. The ligation reaction was incubated for 1 hr at 16°C. Incubating the reaction mixtures at 65°C for 15 minutes inactivated the ligase. The incompletely digested template was destroyed by incubating the ligation mixture with 1000U of Bbs I in NEB2 buffer at 37°C for 2h. This mixture was then used for Bacillus transformation.

### Transformation

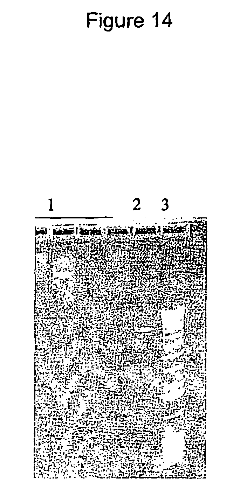

5ul (55ng) of the 440ng ligation was transformed into 200ul of Bacillus competent cells. The cell suspension was shaken vigorously 1 hr at 37°C. One hundred ul of serial dilutions of the cell suspension was plated on selection plates. 129,000 CFU/ug ligation mixtures were obtained, useful for combinatorial library construction. Ligation conditions produced large tandem repeats, which facilitated Bacillus transformation. See Figure 14 (photo). Lane 1 depicts large, low mobility ligation products, Lane 2 depicts mobility of unligated vector. Lane 3 depicts molecular weight standards.

| Lane | DNA | CFU/ug |
|---|---|---|
| 1 | Ligated DNA | 1.3e5 |
| 2 | Linear vector DNA | 0 |
| 3 | 1 KB ladder | |

### Example 8:

### Markerless deletion by insertion

This example demonstrates the deletion of the metB gene of Bacillus. A PCR product was generated from sequences that flank the met B gene. This product and a replicating Bacillus plasmid were co-transformed into the competent Bacillus, and cells resistant to the antibiotic marker on the plasmid were selected. These cells were screened for the metB deletion by methionine auxotrophy and absence of metB sequence from a PCR product.

### Preparation of insert:

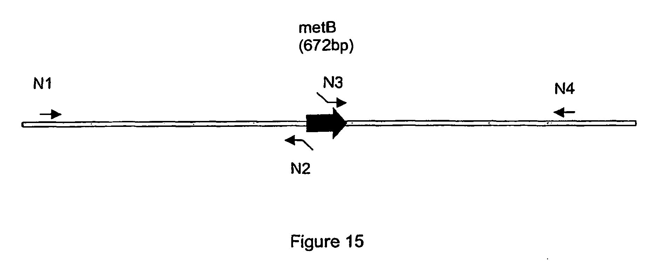

PCR with 100f/r (Primers N1 and N2 in Figure 15) produced a 3958bp and 101f/r (Primers N3 and N4 in Figure 15) produced a 3451 bp. When fused together, a 7409 bp fragment is generated that is deleted for nucleotides 1-621 of metB (full length metB is 672 bp; thus, this is not a full deletion). See Figure **15****.**

| Primers | Primer sequence |
|---|---|
| N1 | AAATGAAGCGCTCCTTCTTTCTTCG (SEQ ID NO: 31) |
| N2 | GCTTCCTTTGATGCGGTAAGAATGTTTACGTGCCACCTCCATTATTTCCCCG (SEQ ID NO : 32) |
| N3 | CGGGGAAATAATGGAGGTGGCACGTAAACATTCTTACCGCATCAAAGGAAGC (SEQ ID NO :33) |
| N4 | GAGCTTGCTCAAGAGCCTGATGACA (SEQ ID NO:34) |

The amplification used 0.5uM of primer pairs N1/N2 or N3/N4, 300uM of dNTP, 200 ng Bacillus chromosome DNA, 5U Herculase (Stratagene) and 1x Herculase buffer (Stratagene) in a 50 ul reaction volume.

The amplification parameters were: 94°C for 3min, 94°C for 30sec, 54°C for 30sec, and 68°C for 7.1 min for a total of 30 cycles. PCR products were purified using the QlAquick PCR Purification Kit.

The assembly of the entire 7.4 kb fragment containing the mutagenized maturation site was done using 100 ng of each PCR fragment, 0.5uM each of Primers N1 & N4, 300uM of dNTP, 5U Herculase (Stratagene) and 1x Herculase buffer (Stratagene) in a 100 ul reaction volume. The parameters for the assembly reaction were as follows: 95°C for 30sec, 55°C for 30sec, and 68°C for 7min for a total of 30 cycles. The PCR reaction products were analyzed on an agarose gel.

### Transformation

Transform 500ng of the PCR fusion product along with 50ng of Bacillus replicating plasmid DNA (provides chloramphenicol resistance) into 100uL of hypercompetent Bacillus cells and plated on nutrient agar plates containing chloramphenicol (5γ) plates. The resulting colonies were screened for methionine auxotrophy and PCR for deletion of metB gene. This method produced >900 recombinant deletions per microgram of transformation mix (>6% of chloramphenicol resistant colonies).

## Claims

1. A method of producing a transformed Bacillus, comprising:
(i) selecting a super-competent Pxyl-comK strain of Bacillus;
(ii) producing a DNA construct *in vitro* wherein said DNA construct comprises an incoming sequence of interest, flanked on each side by a homology box, wherein
a) said homology boxes are flanked by non-homologous sequence, or
b) the assembly of said incoming sequence and said homology boxes is multimerized; and
(iii) directly transforming said Pxyl-comK strain of Bacillus with said DNA construct such that the DNA construct becomes integrated into a chromosome of said Bacillus.

2. The method of claim 1, wherein said DNA construct comprises homologous DNA selected from the group consisting of wild-type, mutagenized and modified DNA.

3. The method of claim 1, wherein said DNA construct comprises heterologous DNA selected from the group consisting of wild-type, mutagenized and modified DNA.

4. The method of claim 1, wherein said DNA construct is a non-plasmid DNA construct.

5. The method of claim 1, wherein the DNA construct is produced without the use of a shuttle vector or an intermediate host.

6. The method of claim 1, further comprising the steps of selecting a target sequence in a chromosome of said competent microorganism, and increasing the homology between said target sequence and said DNA construct.

7. A method of directing the evolution of a sequence in the Bacillus host cell chromosome, comprising:
(i) *in vitro* mutagenesis of a DNA construct wherein said DNA construct comprises an incoming sequence of interest, flanked on each side by a homology box, wherein
a) said homology boxes are flanked by non-homologous sequence, or
b) the assembly of said incoming sequence and said homology boxes is multimerized,
(ii) direct transformation of the mutagenized sequence into a competent Bacillus host cell, which is a Pxyl-comK strain of Bacillus
(iii) screening for, or selection of, mutants possessing or exhibiting a desired property, and
(iv) repeating steps (i)-(iii) for one or more rounds.

8. The method of claim 7, carried out so as to evolve single-copy genes of a competent Bacillus strain.

9. A method for constructing a sequence of interest at a target sequence of a selected microorganism, wherein said target sequence includes a residing marker, said method comprising the steps of:
(i) assembling a DNA construct in vitro comprising an incoming sequence, a selectable marker, and two flaking sequences which are homologous to sequences of said target sequence, wherein
a) the construct further comprises non-homologous outer flanking sequences, or
b) the assembly of said incoming sequence, selectable marker and flanking sequence is multimerized, and wherein said selectable marker of the DNA construct is different than the residing marker of the microorganism;
(ii) directly transforming said microorganism with the DNA construct under conditions permitting the incoming sequence and selectable marker to inactivate the residing marker, and selecting for transformants that include the selectable marker;
(iii) repeating steps (i) and (ii) wherein with each repetition of said steps the DNA construct comprises a selectable marker different from the selectable marker in the previous step and the selectable marker of said previous step acts as the residing marker in said microorganism, wherein said microorganism is a super-competent Pxyl-comK strain of Bacillus.

10. The method of claim 9, further comprising, after step (ii), the step of: testing the transformants for loss of the residing marker, thereby verifying that the construct was incorporated into the correct locus of the chromosome.

## Patentansprüche

1. Verfahren zur Herstellung eines transformierten Bacillus, umfassend:
(i) das Auswählen eines super-kompetenten Pxyl-comK-Stammes des Bacillus;
(ii) das Herstellen eines DNA-Konstruktes *in vitro*, wobei das DNA-Konstrukt eine eintretende Sequenz von Interesse umfasst, die auf jeder Seite durch eine Homologie-Box flankiert ist, wobei a) die Homologie-Boxen durch nicht-homologe Sequenzen flankiert sind, oder b) die Anordnung von der eintretenden Sequenz und den Homologie-Boxen multimerisiert ist; und
(iii) das direkte Transformieren des Pxyl-comK-Stammes des Bacillus mit dem DNA-Konstrukt, so dass das DNA-Konstrukt in ein Chromosom des Bacillus integriert wird.

2. Verfahren von Anspruch 1, wobei das DNA-Konstrukt homologe DNA umfasst, die aus der Gruppe gewählt ist, welche aus Wild-Typ-, mutagenisierter und modifizierter DNA besteht.

3. Verfahren von Anspruch 1, wobei das DNA-Konstrukt heterologe DNA umfasst, die aus der Gruppe gewählt ist, welche aus Wild-Typ-, mutagenisierter und modifizierter DNA besteht.

4. Verfahren von Anspruch 1, wobei das DNA-Konstrukt ein nicht-Plasmid-DNA-Konstrukt ist.

5. Verfahren von Anspruch 1, wobei das DNA-Konstrukt ohne die Verwendung eines Shuttle-Vketors oder eines intermediären Wirtes hergestellt wird.

6. Verfahren von Anspruch 1, ferner umfassend die Schritte des Auswählens einer Zielsequenz in einem Chromosom des kompetenten Mikroorganismus und des Erhöhens der Homologie zwischen der Zielsequenz und dem DNA-Konstrukt.

7. Verfahren des Steuerns der Evolution einer Sequenz in dem Bacillus-Wirtzellchromosom, umfassend:
(i) die *in* vitro-Mutagenese eines DNA-Konstruktes, wobei das DNA-Konstrukt eine eintretende Sequenz von Interesse umfasst, die auf jeder Seite durch eine Homologie-Box flankiert ist, wobei a) die Homologie-Boxen durch nicht-homologe Sequenz flankiert sind, oder b) die Anordnung von der eintretenden Sequenz und der Homologie-Boxen multimerisiert wird,
(ii) die direkte Transformation der mutagenisierten Sequenz in eine kompetente Bacillus-Wirtszelle, welche ein Pxyl-comK-Stamm von Bacillus ist,
(iii) das Screenen für oder Auswahl von Mutanten, die ein erwünschtes Verhalten besitzen oder zeigen, und
(iv) das Wiederholen der Schritte (i)-(iii) eine oder mehrere Runden.

8. Verfahren von Anspruch 7, das so ausgeführt wird, dass Einzel-Kopie-Gene eines kompetenten Bacillus-Stammes entstehen.

9. Verfahren zur Konstruktion einer Sequenz von Interesse an einer Zielsequenz von einem ausgewählten Mikroorganismus, wobei die Zielsequenz einen innewohnenden Marker einschließt, wobei das Verfahren die folgenden Schritte umfasst:
(i) das Zusammenbauen eines DNA-Konstruktes *in vitro,* umfassend eine eintretende Sequenz, einen selektierbaren Marker und zwei flankierende Sequenzen, welche zu Sequenzen der Zielsequenz homolog sind, wobei a) das Konstrukt ferner nicht-homologe äußere flankierende Sequenzen umfasst, oder b) die Anordnung von der eintretenden Sequenz, dem selektierbaren Marker und der flankierenden Sequenz multimerisiert ist, und wobei der selektierbare Marker des DNA-Konstruktes sich von dem innewohnenden Marker des Mikroorganismus unterscheidet;
(ii) das direkte Transformieren des Mikroorganismus mit dem DNA-Konstrukt unter Bedingungen, die es der eintretenden Sequenz und dem selektierbaren Marker ermöglichen, den innewohnenden Marker zu inaktivieren, und Selektieren bezüglich Transformanten, welche den selektierbaren Marker einschließen;
(iii) das Wiederholen der Schritte (i) und (ii), wobei mit jeder Wiederholung der Schritte das DNA-Konstrukt einen selektierbaren Marker umfasst, der sich von dem selektierbaren Marker in dem vorhergehenden Schritt unterscheidet, und der selektierbare Marker des vorhergehenden Schrittes als innewohnender Marker in dem Mikroorganismus wirkt, wobei der Mikroorganismus ein superkompetenter Pxyl-comK-Stamm von Bacillus ist.

10. Verfahren von Anspruch 9, ferner nach dem Schritt (ii) den folgenden Schritt umfassend: das Testen der Transformanten in Bezug auf den Verlust des innewohnenden Markers, wodurch verifiziert wird, dass das Konstrukt in den korrekten Ort des Chromosoms eingebaut worden ist.

## Revendications

1. Procédé de production d'un Bacillus transformé, comprenant :
(i) la sélection d'une souche Pxyl-comK supercompétente de Bacillus ;
(ii) la production d'une construction ADN *in vitro* dans lequel ladite construction ADN comprend une séquence entrante d'intérêt, encadrée sur chaque côté par une boîte d'homologie, dans laquelle
a) lesdites boîtes d'homologie sont encadrées par une séquence non homologue, ou
b) l'assemblage de ladite séquence entrante et desdites boîtes d'homologie est multimérisé ; et
(iii) la transformation directe de ladite souche Pxyl-comK de Bacillus avec ladite construction ADN de telle façon que la construction ADN s'intégre dans un chromosome dudit Bacillus.

2. Procédé de la revendication 1, dans lequel ladite construction ADN comprend de l'ADN homologue choisi dans le groupe consistant en de l'ADN modifié, mutagénisé et de type sauvage.

3. Procédé de la revendication 1, dans lequel ladite construction ADN comprend de l'ADN hétérologue choisi dans le groupe consistant en de l'ADN modifié, mutagénisé et de type sauvage.

4. Procédé de la revendication 1, dans lequel ladite construction ADN est une construction ADN non plasmidique.

5. Procédé de la revendication 1, dans lequel la construction ADN est produite sans l'utilisation d'un vecteur navette ou d'un hôte intermédiaire.

6. Procédé de la revendication 1, comprenant en outre les étapes de sélection d'une séquence cible dans un chromosome dudit microorganisme compétent, et d'amélioration de l'homologie entre ladite séquence cible et ladite construction ADN.

7. Procédé de conduite de l'évolution d'une séquence dans le chromosome de la cellule hôte de Bacillus, comprenant :
(i) la mutagenèse *in vitro* d'une construction ADN dans lequel ladite construction ADN comprend une séquence entrante d'intérêt, encadrée sur chaque côté par une boîte d'homologie, dans laquelle
a) lesdites boîtes d'homologie sont encadrées par une séquence non homologue, ou
b) l'assemblage de ladite séquence entrante et desdites boîtes d'homologie est multimérisé,
(ii) la transformation directe de la séquence mutagénisée en une cellule hôte compétente de Bacillus, qui est une souche Pxyl-comK de Bacillus
(iii) le criblage, ou la sélection, de mutants possédant ou présentant une propriété souhaitée, et
(iv)la répétition des étapes (i) - (iii) pour un ou plusieurs essais.

8. Procédé de la revendication 7, réalisé afin de développer des gènes non répétitifs d'une souche de Bacillus compétent.

9. Procédé de construction d'une séquence d'intérêt à une séquence cible d'un microorganisme sélectionné, dans lequel ladite séquence cible inclut un marqueur résident, ledit procédé comprenant les étapes de :
(i) assemblage d'une construction ADN in vitro comprenant une séquence entrante, un marqueur génétique, et deux séquences adjacentes qui sont homologues des séquences de ladite séquence cible, dans lequel
a) la construction comprend en outre des séquences adjacentes extérieures non homologues, ou
b) l'assemblage desdits séquence entrante, marqueur génétique et séquence adjacente est multimérisé, et dans lequel ledit marqueur génétique de la construction ADN est différent du marqueur résident du microorganisme ;
(ii) transformation directe dudit microorganisme avec la construction ADN dans des conditions permettant à la séquence entrante et au marqueur génétique d'inactiver le marqueur résident, et sélection des transformants qui incluent le marqueur génétique;
(iii) répétition des étapes (i) et (ii) dans lequel avec chaque répétition desdites étapes, la construction ADN comprend un marqueur génétique différent du marqueur génétique de l'étape précédente et le marqueur génétique de ladite étape précédente agit comme le marqueur résident dans ledit microorganisme, dans lequel ledit micro-organisme est une souche Pxyl-comK supercompétente de Bacillus.

10. Procédé de la revendication 9, comprenant en outre, après l'étape (ii), l'étape de : contrôle des transformants quant à la perte du marqueur résident vérifiant, de ce fait, que la construction a été incorporée dans le bon locus du chromosome.
